# EUROPEAN PATENT SPECIFICATION

(11) **EP 3 384 416 B1**
(45) Date of publication and mention of the grant of the patent: **15.03.2023**
(21) Application number: 16871184.4
(22) Date of filing: 03.05.2016
(51) Int. Cl.: G06Q 10/10, G16H 10/20, G16H 30/40, G16H 15/00, G06T 7/00, G06F 16/51

(54) **SYSTEM AND METHODS FOR DISPLAYING MEDICAL INFORMATION**
SYSTEM UND VERFAHREN ZUR ANZEIGE VON MEDIZINISCHEN INFORMATIONEN
SYSTÈME ET PROCÉDÉS D'AFFICHAGE D'INFORMATIONS MÉDICALES

(30) Priority: 30.11.2015 US 201562260724 P
(43) Date of publication of application: 10.10.2018
(73) Proprietor: ASCEND HIT LLC, Lisle, Illinois 60532 (US)
(72) Inventor: SOBLE, Jeffrey, Lisle, Illinois 60532 (US); ROBERGE, James, Lisle, Illinois 60532 (US)
(74) Representative: Brunazzi, Stefano
(86) International application number: PCT/US2016/030596
(87) International publication number: WO 2017/095473

(56) References cited:
- US-A1- 2010 131 283
- US-A1- 2012 035 963
- US-B2- 8 150 857

## Description

### CROSS REFERENCE TO RELATED PATENTS

This application claims the benefit of U.S. Provisional Patent Application No. 62/260,724 filed November 30,

### FIELD OF THE INVENTION

The present invention relates generally to a system and methods for the display of medical information. More specifically, the invention is directed to a system and methods by which information regarding a subject, including that which may be characterized as images or data, may be analyzed and processed - such as according to one or more deconstruction steps appropriate for a clinical ontology chosen by the user in order to, for example, classify, identify and isolate patterns, sets, structures, features, or attributes within the information - and made accessible such as through r the entry of one or more selections - of a topic, heading, and subheading within a medical report template developed according to the same clinical ontology -, and, by the entry of such one or more selections, developed into an efficient display. Advantageously, by reviewing the efficient display t developed through the use of the present invention, a user can prepare a medical report for a subject and complete a clinical study more thoroughly and more efficiently.

### BACKGROUND

Obtaining access to, reviewing, and interpreting images are key tasks that must be accomplished in order to conduct a clinical study for and render medical care to a patient in many situations. To accomplish these tasks more efficiently, health care workers have come to develop a generally standardized approach.

Certain known standardized approaches begin with the capture of one or more of the images that the health care worker determines are needed to conduct a clinical study. The objective of the selected clinical study may be to determine the health of a patient or to determine the medical cause of a certain condition that the patient is experiencing, or to achieve some other goal. The images that are captured are then organized according to a pattern that is generally defined by a standard protocol or just the image acquisition sequence. The health care worker may then conduct a review and analysis of the images and, from that review and analysis, record observations and opinions in a medical report. The completion of the report may represent the completion of one part or the entire medical study.

The replacement of analog materials and systems with digital systems has made it easier and quicker for health care workers to complete reviews of images and produce a medical report. However, other than the change of the expression of information in digital form, the workflow for reviewing and interpreting medical images and producing a medical report has largely changed little over time.

For example, the system that health care workers use to organize the images that have been captured has largely not changed. The traditional approach often involved the use of what are termed "hanging protocols". The term "hanging protocols" originally meant the arrangement of developed analog film images on a light box or the hanging of such film images on a film alternator. With the adoption of digital film-based systems, the term now refers to the pre-determined settings that define which and how digital film images are displayed. For example, standard x-ray images have standard projections which are displayed in a reproducible layout, ultrasound images are typically displayed in the temporal sequence, and CT or MRI images are often displayed by orientation in space (axial, coronal, sagittal). Often, health care workers use a "picture archiving and communication system" ("PACS") and image review workstation to organize and display images.

With respect to either type of display, film or digital, the goal of a hanging protocol is to present specific types of captured images in a consistent manner thereby reducing the number of adjustments that the health care worker must manually make in order to conduct a certain review. Specifically with respect to digital images, certain versions of hanging protocols allow a medical practitioner to display a particular set of images in a particular sequence or order on a screen or interface and/or across multiple display screens or interfaces. The typical sequence organizes the images according to time at which each of the images is acquired such that the first acquired image may be placed first followed by later acquired images in a grouping of "thumbnail"-sized images. While hanging protocols can be varied by modality, body part, department, and the personal preference of the health care worker, hanging protocols cannot be customized based on the specifics of an individual patient's pathology or even the clinical indication for the imaging study. Many computerized medical image review systems include a set of default hanging protocols or allow users to define their own.

Also, the way in which health care workers record their observations and opinions in the form of a medical report has largely not changed. The traditional approach of medical reporting involved the use of largely standardized forms - at first, in analog form - to record observations and state opinions based on a review of the image or images. Currently, such standardized forms include a text-based report - that may be completed, for example, using dictation and voice recognition with little or no coded data - or a structured (data-driven) report - that may be completed, for example, using coded data elements to produce a report narrative.

One advantage of using the conventional approach to organizing images and reporting the observations made and opinions formed from a review of these images is predictability: a physician such as a radiologist or cardiologist who reads studies of medical images typically reads a collection of similar studies all at one time (such as a group of chest x-rays, echocardiograms, or CT scans). Once the study type is defined, the user knows what images to expect, how the images will be organized and displayed, and largely what form the user will use to generate the medical report. For instance, when asked to read a coronary angiogram or echocardiogram, the cardiologist will traditionally play one or more cineloops. A "cineloop" is a series of images captured at a high frame rate and stored such that the sequence of individual frames can be played with a PACS as a movie. From this review, the cardiologist will prepare a medical report - by either dictating the substance of the report (for transcription or voice recognition) or use computer structured data from a computer program developed for the purpose. Similarly, a radiologist will review a specific type of CT scan, such as a chest CT, from the same set of views in the same order for each case, and go through a very similar process of image review for each case.

Many disadvantages are associated with using the traditional approach to generate a medical report based on image review.

One disadvantage associated with the traditional approach is that only a single set of images are organized and provided for review at one time. The traditional approach does not facilitate the identification of a wider range of images that may be relevant and that may appear in different sets and organize those identified images into a group that can be readily reviewed by a health care worker. The sets in which the relevant images may be found may be the result of not only generally contemporaneous imaging but also historical imaging in which the historical images reside in archives. In order to be able to make use of such images, traditional image review and reporting systems often require the health care worker to access and review or scan a complete series of images in its entirety. Traditional approaches typically do not facilitate the efficient identification of and access to specific images or subsets of images within those relevant retained images sets.

Another disadvantage is that the traditional approach typically organizes and provides a single set of images captured through the use of a single modality. The traditional approach does not facilitate the identification and organization of various series of images taken through the use of different modalities into a group that can be readily reviewed by a health care worker. As an example, in order to analyze right ventricular function by echocardiography, a health care worker may decide it is necessary to see the right ventricle from multiple views. These views may exist within separate image series. It may be necessary to review Spectral Doppler images showing pulmonary artery pressure and color flow Doppler images showing tricuspid regurgitation. The most relevant images may reside within disparate locations of a very large data space. For example, the most relevant images may be discovered within 100 or more "cineloops". Reviewing such a number of cineloops in order to find a specific feature can be particularly time consuming given that each cineloop is typically captured at such a high rate of speed that more images are acquired than is needed.

An additional limitation to the traditional approach is that the large body of information that may be relevant to a subject is not organized such that a health care worker can readily access it during the course of reviewing the images and preparing the medical report. Such a large body of relevant information may include various types of data that may be relevant to a subject. Such data may include that which is termed for purposes of this application as "quantitative data". Quantitative data is that which is produced for example, through the use of analytic, diagnostic, and monitoring equipment, some equipment of which may utilize software. Quantitative data may be not only that which is produced for a particular patient but also, for example, anonymized data developed for one or more other individuals that may be useful to a health care worker in order to compare with and place the patient's data in perspective. Data may include also "quantitative image data" - defined for purposes of this application as the information, content, and measurements drawn from one or more images - and "qualitative data" - defined for purposes of this application as the observations, opinions, or findings of one or more health care workers based on their review of other data, images, or the quantitative image data. Traditionally, the data that may be relevant to the clinical study being conducted for a particular subject is separately stored - such as "islands" or "silos" of data - within larger sets of data. Because the data relevant to and the image or images captured for a subject are not organized for efficient and rapid access by a health care worker, in order to complete a medical report for a subject for whom an image or images have been captured, a health care worker must access what may be a very large data space, search the information retained there, identify the most relevant stored data and other images, review the identified stored data and images, record observations of the newly captured image or images possibly in light of information provided by the retained data and images, and, to complete the clinical study, prepare the medical report. Conducting all such steps may be a time consuming task.

Because the traditional approach of organizing a subject's information and images does not permit efficient access to and the review of all information - current and historical - regarding the particular anatomy, physiology, or pathology that is of interest for a subject, a health care worker cannot easily conduct what is termed a "longitudinal study" or a "longitudinal comparison" for the subject. A longitudinal study is a review that seeks to determine whether the function of an anatomical structure has changed over a period of time. Typically to conduct the longitudinal study, current and historical series of imaging studies within the same modality or across modalities must be examined. However, each such imaging study may contain multiple image data sets, quantitative image data sets, and qualitative data sets relating to the anatomical structure of interest. Hanging protocols are unable to efficiently display images or data that were captured during multiple acquisitions, and/or as a result of multiple longitudinal studies within a single modality, and/or through the use of multiple modalities. Navigating between and within studies to locate the relevant data is known as an inefficient process.

Ultimately, because of the difficulties in identifying and accessing older, yet what may be highly relevant information, a health care worker may not have the time to review such information. The medical report that the health care worker prepares as a result may be more limited in perspective. US 2012/035963 A1 discloses a text extraction unit that extracts, structures and encodes clinical information in patient records, a reasoning engine that analyzes clinical information and identifies a reason for a medical report generation request and a reasoning engine that analyzes patient images and suggests a pre-generated report template based on identified reason. US 2010/131283 A1 discloses a workflow assistance for user development of clinical content for execution via a clinical interface. US 8150857 B2 discloses a template identifying subject matter specific settings, and the settings comprise a default ontology or taxonomy.

Clearly, there is a need for a system and methods by which a wide range of information, including new and historic images and data relevant to the condition of a patient, can be efficiently identified, organized, and made available for access and analysis so that a medical report can be prepared and a clinical study completed more efficiently. The present invention satisfies these demands.

### SUMMARY OF THE INVENTION

The present invention relates generally to a system and methods for displaying medical information. More specifically, the invention is directed to a system and methods by which information, such as one or more images and one or more data sets, may be identified, analyzed, and organized and a report template produced, each according to a chosen clinical ontology, such that by the selection of the appropriate topic, heading, and subheading of the template, a user may obtain access to the appropriate information and be able to prepare a medical report and complete a clinical study for a subject more efficiently.

Certain preferred embodiments of the present invention include a template development component - through the use of which a medical report template may be prepared that is generally customized for the type of medical study to be performed - and an information identification component - through the use of which information may be obtained, and/or identified, analyzed, and organized for access through the use of the medical report template. The information may include image information drawn from one or more images and data information developed from one or more sets of data and linked to the image information. By organizing the template and the information according to the same chosen clinical ontology, an efficient display of the information may be formed. The efficient display of information formed through the use of the present invention is termed also an "adaptive display". The adaptive display may be distributed through a network to one or more display outputs to which are connected one or more displays on which the adaptive report may be shown to one or more users. An "adaptive display" for purposes of this application is one in which at least certain or all the information that has been obtained for a subject is organized and made accessible according to the same clinical ontology on which the template report is based so that a user may be provided with the corresponding appropriate information simply by selecting the heading or subheading of the template report. Through the use of the template report and the adaptive display tool, the user can conduct and report the results for a clinical study more efficiently.

Certain embodiments of the template development component allow the user to produce a report template customized for a subject by permitting the user to define the clinical study that the user wishes to conduct for a subject and the clinical ontology that the user wishes to use to identify and categorize the information relevant to the subject which the user wishes to consider for purposes of conducting the clinical study.

Certain embodiments of the system and methods according to the present invention include an information identification component through the use of which information from one or more sources may be accessed and identified and be available for processing such that elements drawn from the sourced information and content relevant to the sourced information may be developed and organized according to the chosen clinical ontology. The information identification component may use either or both an image identification component - by which "image information" may be drawn by the "deconstruction" of the one or more of the images captured for a subject - and a data identification component - by which "data information" may be drawn by a similar "deconstruction" of certain or all the data collected or obtained for a subject. The information that may be deconstructed may be, for example, also the anonymized information obtained from one or more other individuals and used to place the information obtained for a subject in context.

Certain embodiments of the image identification component may include an image classification component, an image segmentation component, and an attribute identification component by which image information may be developed through the "deconstruction" of the one or more images captured for a subject according to the clinical ontology - also termed "clinical parameters" for purposes of this application - chosen for a subject.

Embodiments of the image classification component may be used to categorize the overall properties of a complete image. One frame within the series of frames that form a cineloop may constitute a complete image. Some categories in which an image may be placed concern, for example, the modality used to produce the image (e.g., ultrasound) and the "view" which the image shows (e.g., long axis, short axis, AP, or lateral). Examples of categorization information include the PA image of a PA and Lateral chest x-ray, the 4 chamber view of an echocardiogram, an axial CT image at the level of the carina, or a sagittal MRI image of the brain at the level of the pituitary.

Embodiments of the image segmentation component may be used to isolate one or more structures that may appear within an image. Examples of structure isolation information that may be developed through the use of the image segmentation component include the left cardiac border on a chest x-ray, the anterior leaflet of the mitral valve on an echocardiogram, the pituitary gland on a MRI, or a lung mass in a chest CT. For purposes of this application, structure isolation information may be anatomic or functional (such as mitral regurgitation by Doppler), and may be 2, 3, or 4-dimensional.

Embodiments of the attribute identification component may be used to extract attribute information from a segmented image, such as the size, function, or pathologic characteristics (e.g., valve regurgitation) of an identified structure.

Certain embodiments of the data identification component are configurable to deconstruct a wide range of data collected or obtained for a subject, including current and historical data, for example, according to the clinical parameters chosen for a subject.

In certain embodiments, as the process of deconstructing the information into, for example, image information and data information proceeds, the deconstructed information may be stored, organized, made accessible, and "assembled" such as in one or more information retention elements - according to the clinical parameters to develop ontology-defined information. Embodiments of the present invention may include an information resource that facilitates such processing by a chosen organizational ontology and in which at least the ontology-defined information may be stored and made accessible such as in one or more information retention elements such that a user - by entering a selection, for example, of a heading or subheading of the template report - may easily obtain the corresponding relevant information.

Additional embodiments of the present invention allow a user to configure the adaptive display tool such that the user may be informed of not only contemporary information but also historical information that is appropriate for the selected heading or subheading of the template report. Advantageously, by knowing the scope of, having ready access to, and being able to review this combination of such contemporary and historical information, a user can more quickly conduct a "longitudinal study" (or "longitudinal comparison") - that is, a comparative analysis of a certain aspect of a subject or subjects condition over time - and possibly conclude whether, for example, the patient's condition has improved, worsened, or remained the same. Information needed to conduct such a longitudinal comparison may be stored in the information resource established for the patient.

Added embodiments of the present invention provide an adaptive display tool - such as one made accessible to one or more users through a network to which are connected one or more display outputs connected to a display for displaying the tool, certain embodiments of which are presented as a computer display user interface with which a user may interact through one or more engagement components. By the use of the one or more engagement components, a user may instruct a processor to perform one or more computer-implemented actions. One such engagement component permits a user to interact with the interface and instruct the processor with respect to one or more features of the system by voice. Another engagement component permits entry through "clicking" on a feature shown on the computer user interface computer display. Others permit interaction through a keyboard key, button, or hand control device that includes information entry features in which a user may navigate through, make selections, and enter data in the tool such as through voice.

Other embodiments of the present invention may combine features of the adaptive display tool with more traditional image analysis systems to form a hybrid display system and methods. One embodiment of such an adaptive hybrid display system may permit a user to enter a selection to obtain the image information or data information relevant to a heading or subheading of a medical template while conducting a review of images largely through the use of traditional techniques - such as hanging protocols or standard sequential image review tools. For example, the hybrid display user interface may permit an echocardiographer to choose to read cardiac ultrasound information in standard sequential fashion but, during the course of that reading, enter a selection that displays the image information or data information relevant to the selection (e.g., Aortic Valve). As another example, a radiologist may review an abdominal CT in a traditional fashion - using axial, coronary, and sagittal image planes - and enter a selection through the display user interface that draws information relevant to the selection from an information resource and displays it for review during the course of the clinical study as, for instance, a 3D volume rendering of the liver, pancreas or kidneys when a corresponding heading is selected.

Additional embodiments of the present invention permit a user to choose whether and with respect to which clinical parameters to emphasize during the course of the clinical study. For example, rather than organizing and making available to the user the same image information and data information regardless the patient, this adaptive display direction tool permits the user to select a specific patient and the patient's medical condition. Information and data particular for that condition will be shown in preference to other information. For example, for a patient with a known medical condition of the mitral valve - such as a rheumatic heart disease -, a user may enter the appropriate selection and, for the relevant clinical parameters, additional information may be provided - such as information regarding mitral thickness, velocity, and stenosis. The user interface may emphasize that the user review this additional information - such as by preventing the user from progressing through the clinical study and preparing a medical report - until the user has demonstrated that the user has reviewed the emphasized information.

Added embodiments of the present invention facilitate the creation of associations of the information such as the association of historic image information and/or contemporary image information and data information that is being produced during the course of the current clinical study. For example, the adaptive display may permit a user to enter data in heading or subheadings of a template and choose to what image information - historic and/or contemporary - to link the data information. The adaptive association display tool may also be configurable to permit the association linkages to be created automatically such as based on past activities of the user and/or the past activities of a larger user population with respect to similar patients and/or based on the past activities of the user with the patient that is the subject of the current medical study.

### BRIEF DESCRIPTION OF THE DRAWINGS

Embodiments are illustrated by way of example and not limitation in the figures in the accompanying drawings, in which like references indicate similar elements and in which:
FIG. 1A is a flow chart illustrating the operation of one preferred embodiment of the present invention;
FIG. 1B is a flow chart illustrating the operation of one preferred embodiment of the present invention;
FIG. 1C is a flow chart illustrating the operation of one preferred embodiment of the present invention;
FIG. 2A illustrates a prior art display of images organized sequentially in acquisition order;
FIG. 2B illustrates components of a prior art standard report template in which certain information has been entered;
FIG. 3A illustrates one preferred embodiment of a graphical user interface by which a user may configure and interact with the system formed in accordance with the present invention;
FIG. 3B illustrates another preferred embodiment of a graphical user interface by which a user may configure and interact with the system formed in accordance with the present invention;
FIG. 3C illustrates an additional preferred embodiment of a graphical user interface by which a user may configure and interact with the system formed in accordance with the present invention;
FIG. 3D illustrates an additional preferred embodiment of a graphical user interface by which a user may configure and interact with the system formed in accordance with the present invention;
FIG. 4 illustrates an exemplary computer system that may be used to implement the methods according to the present invention; and
FIG. 5 illustrates a cloud based system that may be used to implement the methods according to the present invention.

### DETAILED DESCRIPTION OF EMBODIMENTS OF THE INVENTION

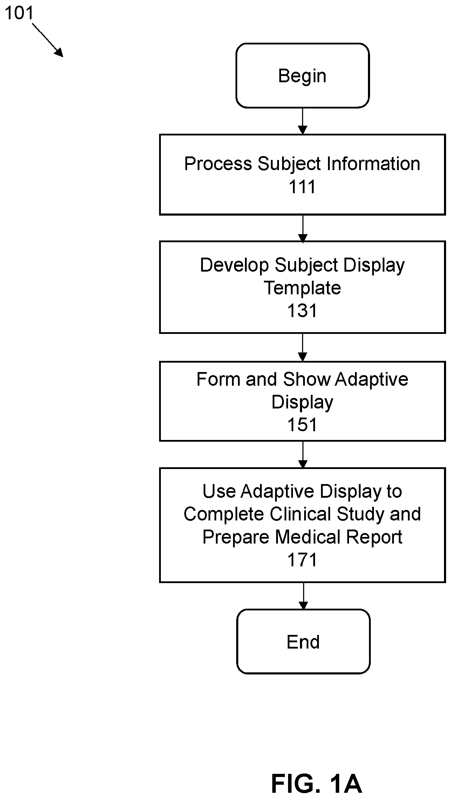

One preferred embodiment of a system 101 according to the present invention by which information may be processed for and an adaptive display developed and used in order to complete and report the results of a clinical study is shown in FIG. 1A.

More specifically, the embodiment of the system 101 includes a processing step in which information that may be relevant to a subject - such as that provided through one or more images and data - can be received and processed 111. The information may wholly be or include that which was developed from the work conducted on an individual patient - also termed "subject" for purposes of this application - but also the information that was developed from the work conducted on one or more other patients but which may be relevant to the subject. Certain embodiments of the processing step 111 may use an information identification component to obtain information from one or more sources that may be processed such that elements may be drawn and content relevant to the subject may be developed, organized, and made accessible according to selected clinical parameters. The information identification component may include an image identification component by which image information may be developed by, for example, categorizing the properties, identifying and isolating the structures, and extracting the attributes of that which appears in the one or more images. Alternatively, or in addition to the image identification component, the information identification component may include a data identification component by which data information may be developed through a similar "deconstruction" of certain or all the data collected or obtained such as for a subject. During the course or at the completion of the deconstruction of the information into, for example, image information and/or data information, the processing step may include organizing and assembling the deconstructed information according to the selected clinical parameters. Embodiments of the present invention may include an information resource in which at least the assembled information may be stored and made accessible to a user such that the user - by entering a selection, for example, of a heading or subheading of the template report - may easily obtain the information assembled according to the clinical parameter that matches to the selected template heading or subheading. The assembled information may be received and stored in the information resource. The information resource may be partitioned to provide more efficient access.

The system 101 shown in FIG. 1A permits a display template to be developed 131. Certain embodiments of the display template may include headings and subheadings that are appropriate for the clinical parameters by which the deconstructed information is assembled. Advantageously, by such compatible organization of the display template, a user's selection of a heading or subheading will provide the user with access to information that is assembled according to the selected heading or subheading. The template may be developed for a specific individual subject or a group of subjects whose medical conditions may be similar.

The system 101 shown in FIG. 1A additionally facilitates the development of an adaptive display 151. Certain embodiments of the adaptive display are made available to a user as an interactive display on a computer user interface. Certain such embodiments permit a user to select a heading or subheading drawn from the display template. Because the heading and subheading of the display template and the assembled information are both organized according to the same clinical concept, a user's selection - such as through the interactive display - of a heading or subheading will provide the user with information assembled in line with that heading or subheading.

Once the adaptive display is formed, the system shown in FIG. 1A facilitates the use of the display to complete and report the results of a clinical study 171. Certain preferred embodiments of the present invention facilitate the distribution of the medical report to one or more end users.

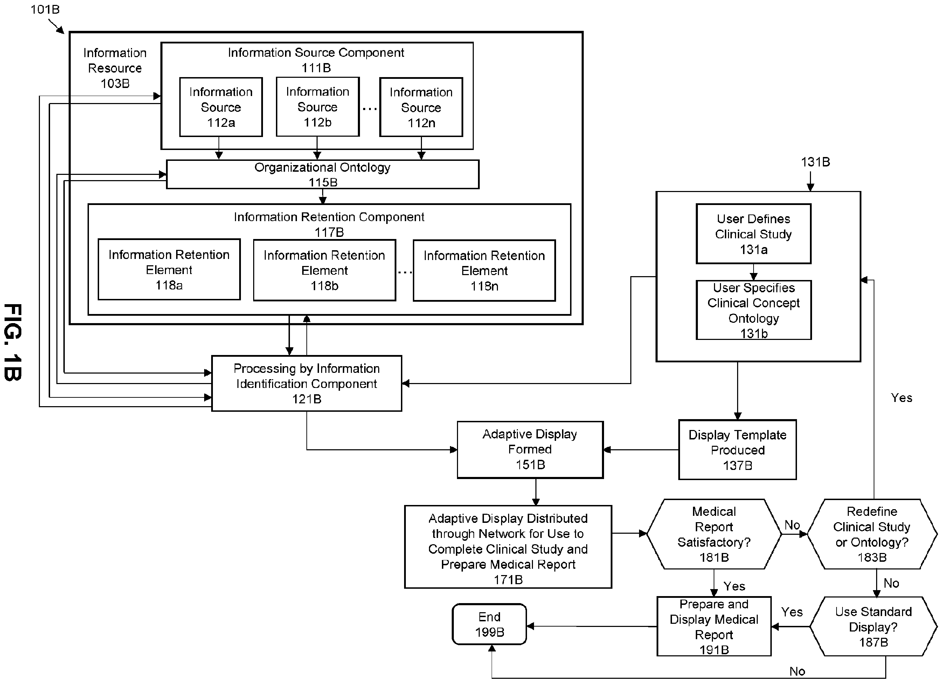

FIG. 1B shows another preferred embodiment of a system 101B according to the present invention. System 101B permits a display template to be developed and information processed according to selected clinical parameters in order to form an adaptive display that may be used in order to complete and report the results of a clinical study.

More specifically, the embodiment illustrated in FIG. 1B includes an information resources component 103B by which the information - such as images and data - that may be used for purposes of conducting the clinical study and preparing the medical report will be sourced and made available for use. The preferred embodiment of the information resources component 103B illustrated in FIG. 1B includes an information source component 111B, an organizational ontology component 115B, and an information retention component 117B.

The information source component 111B of the FIG. 1B embodiment may draw or provide access to information from one or more sources of information, shown as information source elements 112a, 112b, 112n. The information within such source elements 112a, 112b, 112n may be that which is obtained for a specific subject or subjects such as an image or images, data, or a record not otherwise processed by the system 101B. The information within such source elements 112a, 112b, 112n may be that obtained from a clinical study or studies or include additional images, data, and content. The information may be all of the information that may be included within an electronic health record. The information may be graphical or visual representations of data including that obtained from ECG studies or pressure tracings. Such information may be obtained from governmental and public sources or one or more third party providers or developed by or for the user and/or that which is specific to one or more subjects, including historical information developed in advance of the clinical study and stored as archived information, or generally contemporaneous information developed near the time of the clinical study, or new information developed as needed during the course of the clinical study. Additionally, certain of the information may be that which appears in a printed form and has been scanned and is stored in digital form.

The embodiment of the system 101B illustrated in FIG. 1B includes an organizational ontology component 115B by which at least some of the information available through the information source component 111B may be organized according to specific parameters for retention in the information retention component 115B. The parameters by which the information may be organized may be selectable in certain embodiments by the user through the use of the clinical parameters identification component 131B (discussed below) and may be clinical concepts that may be proprietary - such as that developed by or for a business that provides imaging or reporting systems - or public - such as the medical reference terminologies identified by the acronym SNOMED, SNOMED CT, or SNOMED Clinical Terms. The organization of at least some of the information made available through the information source component 111B may facilitate the more expeditious completion of the clinical study and preparation of the medical report.

The information as organized through the use of the organizational ontology component 115B may be stored and made accessible to a user through an information retention component 117B. The information retention component 117B may be partitioned in one or more ways to facilitate the management of the information such as to make it more accessible to a user. In the illustrated embodiment, the information retention component 117B is partitioned to include a plurality of image retention elements 119a, 119b, 119n - in which image-related information and data-related information may be stored. One embodiment by which such storage may be facilitated is through one or more computer memories as described more completely below. The source elements - such as elements 115a, 115b, 115n - may be one or more databases or storage "libraries". Advantageously, the ability of a user to aggregate information in information resources component 109B facilitates efficient searching for information and the selection of content from it by allowing a user to access one resource rather than multiple sources. Embodiments of the system 101B may include an information retention component 117B that includes one or more elements 119a through 119n in which information or the content drawn from it may be segregated or partitioned, such as by a user, and/or according to, for example, the target, type of content, time period, or the metadata identifying, for example, the source of the information, the content that was drawn from it, the date or time of creation or access to the information thereby facilitating even more efficient searching, selection, and use.

The preferred embodiment illustrated in FIG. 1B includes a clinical parameters identification component 131B. The clinical parameters identification component 131B includes a clinical study identification component 131a - by which a user may identify, for example, the subject or subjects of the clinical study, the nature of the clinical study that is to be conducted, and for whom the medical report or reports are to be prepared - and a clinical concept ontology identification component 131b - by which a user may identify, for example, the ontology which will be used to develop the template report and organize the information that is accessed by the user in order to conduct the clinical study. The ontology of clinical concepts may be proprietary - such as that developed by or for a business that provides imaging or reporting systems - or public - such as the medical reference terminologies identified by the acronym SNOMED, SNOMED CT, or SNOMED Clinical Terms. The use of an ontological system to develop the display template and organize the information advantageously can facilitate the management of the complexity of these tasks and allow the clinical study to proceed more expeditiously.

The embodiment of the system 101B shown in FIG. 1B may be used to develop a display template 137B. The display template may include headings and subheadings and overall have an ontological structure that are appropriate for the clinical parameters chosen by the user. The template may be developed for a specific individual or a group of individuals whose medical condition may be similar.

The embodiment of the system 101B shown in FIG. 1B facilitates the processing of the information through the use of an information identification component 121B according to the clinical parameters selected by a user. More specifically, the information identification component 121B may access and process information such as the image information, the data information, and/or other information in order to draw certain elements and content from the sourced information and content, then organize it according to the chosen clinical parameters. The information identification component may include either or both an image identification component through which "image information" prepared by the "deconstruction" of the one or more of the images captured for a subject and a data identification component through which "data information" prepared by a similar "deconstruction" of certain or all the data collected or obtained for a subject is drawn. This deconstructed information may be organized and reassembled - or more simply for purposes of this application "assembled" - according to the clinical parameters. The deconstructed information as well as the assembled information may be stored, for example, within the information retention component117B during the processing stage and subsequently made accessible to a user such as during the use of the adaptive display. To further illustrate the deconstruction of information, one or more images or a set of images may be segregated according to image view, image modality, region of interest segmented such as according to the structure or physiological signal visible within the image or images, a specific image or frame with an image cineloop, a plane of interest through a 3D image set taken at or within a certain range of time, or a 3D volume segmented from a complete 3d or 4D dataset. Advantageously, certain embodiments of the present invention allow a user to access and link other information - including current or historic quantitative data and/or historic image information - to such deconstructed image components in order to place the current images in perspective and permit a more comprehensive medical report to be completed.

The embodiment of the system 101B shown in FIG. 1B permits an adaptive display to be formed 151B by the use of the display template produced in step 137B to obtain the relevant ontology-defined information developed through step 121B. Advantageously, the adaptive display provides the ontology-defined information in the framework and context of the display template. The information processed according to the clinical ontology chosen by the user through the use of the information identification component 121B will be termed "ontology defined information" for purposes of this application.

The adaptive display may then be distributed to a user - such as through a network - for receipt in a computer system and display by the user on one or more digital screens in order to prepare a medical report 171B and complete the clinical study for the subject or subjects. The system 101B may then query the user whether the medical report is satisfactory 181B. If it is, the process is completed 191B. If it is not, the system 101B permits the user to redefine the clinical study or the ontology used to organize the information and the report template.

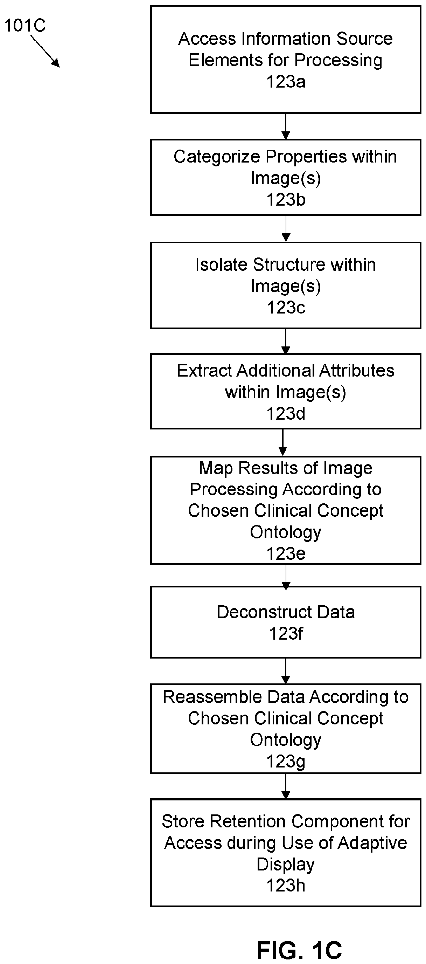

FIG. 1C illustrates an embodiment of an exemplary method 101C by which information may be processed by an embodiment of the information identification component 121B. The method 101C includes steps for the processing of image information and data information. The processing of such image information may occur before or after the data information is processed. In accessing step 123a, the information that may be retained in the information retention component 117B - such as in one or more information source elements 112a, 112b, 112n - is accessed for processing. In categorizing step 123b, embodiments of the information identification component - that include an image classification component - categorize the overall properties of a complete image. In isolating step 123c, embodiments of the information identification component - that include an image segmentation component - isolate one or more structures that may appear within an image. In extraction step 123d, embodiments of the information identification component - that include an attribute identification component - extract attribute information from a segmented image, such as the size, function, or pathologic characteristics (e.g., valve regurgitation) of an identified structure. In mapping step 123e, the results of the image processing are mapped according to the chosen clinical concept ontology. Data may then be deconstructed 123f and reassembled according to the chosen clinical concept ontology 123g. The processed image components and processed data may then be stored - such as in information retention component 117B - for access during use of the adaptive display 123h.

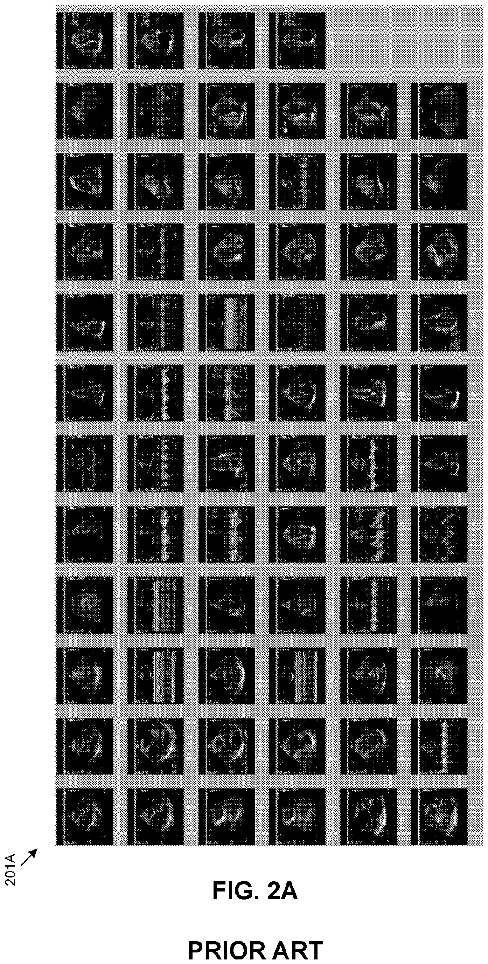

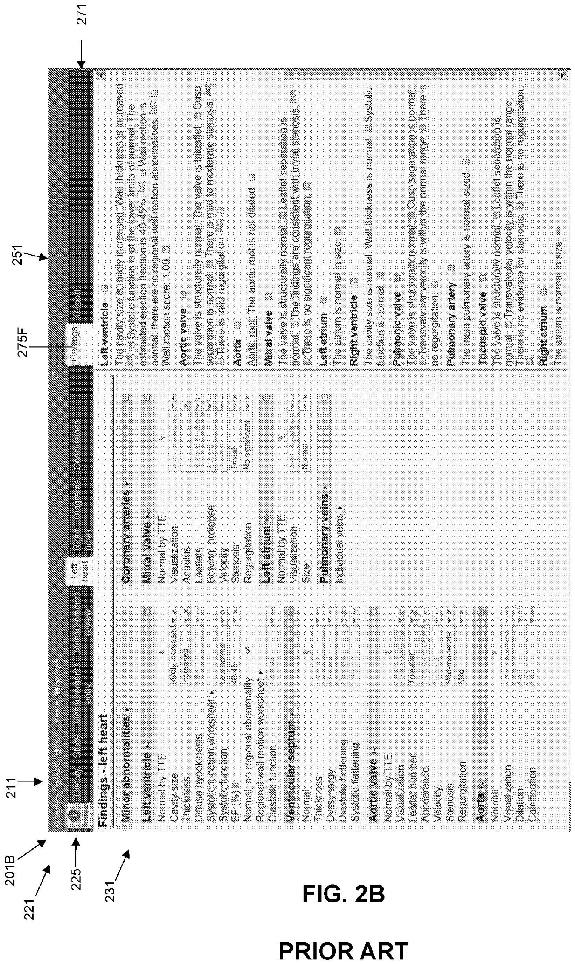

FIG. 2A and FIG. 2B provide information regarding certain traditional forms of displaying medical information. More specifically, FIG. 2A illustrates a prior art display of medical images presented as a grouping 201A. The images within the grouping 201A illustrated in FIG. 2A were captured for a subject during a transthoracic echo study. Each of the images within the grouping 201A are shown in "thumbnail" size and organized sequentially in acquisition order in columns such that the first captured image is positioned in the upper left corner of the display at the top of the left most column and the subsequent captured images arranged below in the same first column and in successive columns. In order to conduct a clinical study for the subject from whom these images were captured, a reviewer would be required to review each of the 70 images within the grouping 201A. From this review, the user may be able to determine which image or images provide the information that the reviewer believes is particular relevant for the subject. A review of some 70 images to determine which images may be useful to direct more attention and analyze for the clinical study is time consuming and prevents a medical report from being produced in the most efficient manner. The present invention seeks to reduce the need for this initial review by displaying to the reviewer those images which match the ontology pre-selected by the reviewer. A clinical study may be completed and a medical report issued in a more time fashion.

FIG. 2B illustrates a known standard display template 201B that has been used, at least in part, to conduct a clinical study for a subject. The illustrated known template 201B includes a data input section 211 and a data and text output section 251.

The data input section 211 of the illustrated known display template 201B includes a data input tab section 221 having a number of specific tabs 225, the engagement of some of which identify to the user the information that the system will permit the user to input and from which a medical report may be generated. The Left heart tab 2125LH is shown in the FIG. 2B template 201B as being engaged. As a result, the user is presented with one or more data input screens 231 relevant to the left heart.

The data and text output section 251 of the standard template 201B shown in FIG.2B includes a data output tab section 271 having a Findings tab 275F the engagement of which displays a narrative for each of the topics for which information was entered through the data input section 211.

To use the standard template 201B shown in FIG. 2B, the user would need a system having two screens on one of which could be displayed the image or images that the user was reviewing and on the second of which could be displayed this template 201B that the user was using to enter the observations were made by separately viewing the relevant images and entering the data and observations in the report. The template 201 provides a more complete narrative section 251 developed from the observation entries.

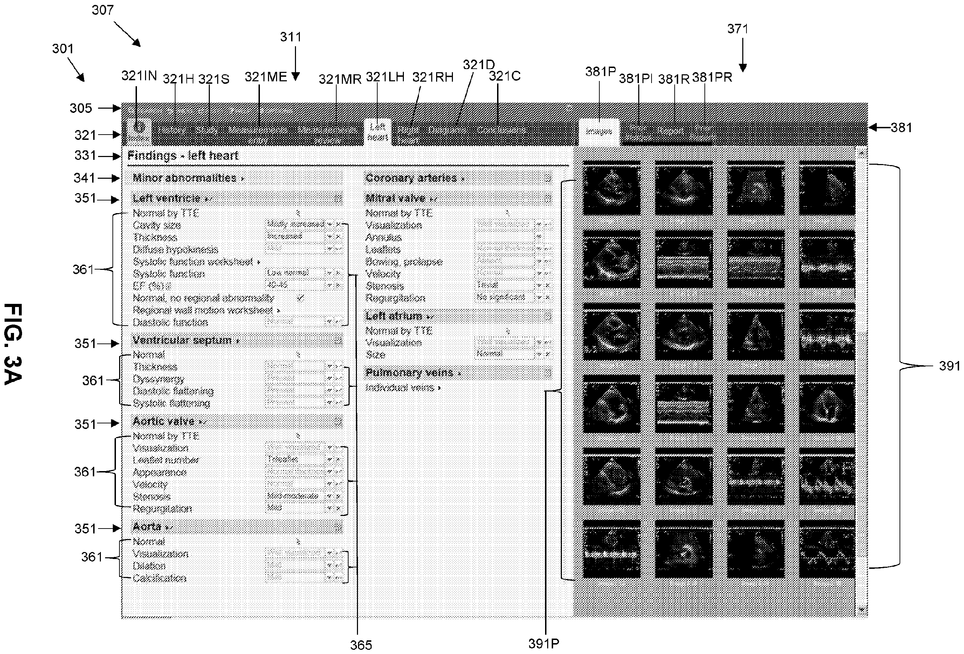

FIG. 3A through FIG. 3D illustrate embodiments of a graphical user interface screen 301 presenting an adaptive display 307 that a user may configure in order that the user may interact with the an embodiment of the system formed in accordance with an embodiment of the present invention. The illustrated embodiments of the adaptive display 307 includes a findings display component 311 and an information display component 371.

FIG. 3A illustrates one embodiment of a user interface screen 301 showing the adaptive display 307 developed according to the present invention. The findings display component 311 of the illustrated embodiment of the adaptive display 307 includes a findings tab section 321 having a plurality of tabs through the engagement of each of which a user may obtain information such as that organized according to topics. The illustrated embodiment of the findings tab section 321 includes an Index tab 321IN, a History tab 321H, a Study tab 321S, a Measurements entry tab 321ME, a Measurements review tab 321MR, a Left heart tab 321LH, a Right heart tab 321RH, a Diagrams tab 321D, and a Conclusions tab 321C.

In the embodiment of the adaptive display 307 illustrated in FIG. 3A, the "Left heart" tab 321LH is shown as being engaged and, by such engagement, a Findings selection section 331 - titled "Findings-left heart" - is displayed to the user. The Findings selection section 331 may display to the user one or more groups of topics, topics, and subtopics. The embodiment of the Findings selection section 331 that is illustrated in FIG. 3A shows the display of one such topic group 341 - titled "Minor abnormalities" - the display of a number of topics 351 - title "Left ventricle", "Ventricular septum", "Aortic valve", "Aorta", "Mitral valve", "Left atrium", and "Pulmonary veins" - and for each of the topics 351, the display of one or more information subtopics 361 and, for each of which , an information entry element 365 in which a user may enter findings developed, for example, by observing the information provided in the information display component 371, such as images and/or text or other data.

The embodiment of the adaptive display 307 illustrated in FIG. 3A includes an information display component 371 having an information display tab section 381 providing a plurality of information display tabs through the engagement of which a user may obtain information. The illustrated embodiment of the display component 371 includes an Images tab 381P, a Prior Images tab 381PI, a Report tab 381R, and a Prior Reports tab 381PR of which the Images tab 381P is shown as being engaged thereby displaying in this embodiment a plurality of echo "thumbnail" images 391P in the information display section 391.

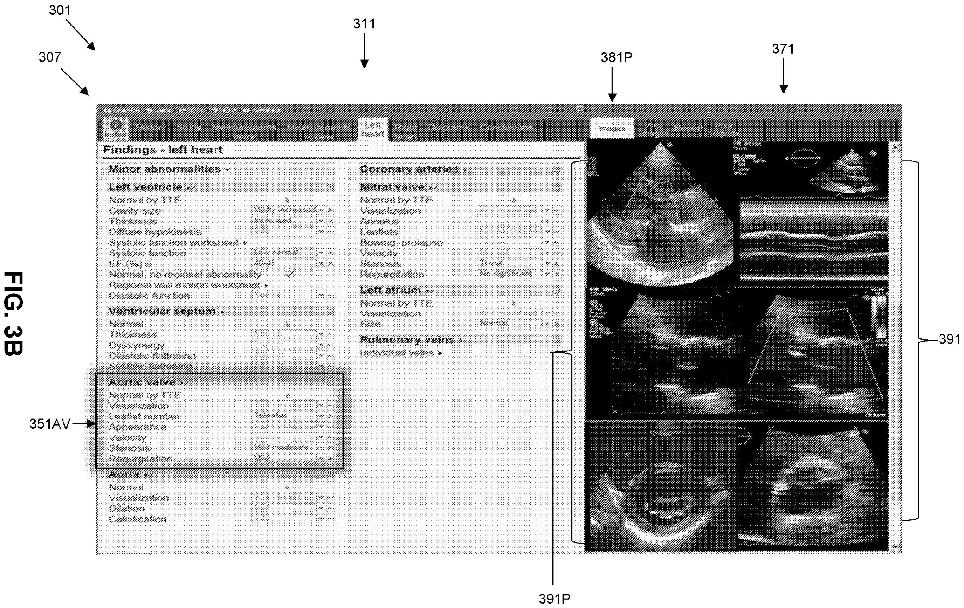

FIG. 3B illustrates the embodiment of the user interface screen 301 illustrated in FIG. 3A in which the topic identified by the heading "Aortic valve" 351AV is shown as being selected by the user (shown by the shading around the "Aortic valve" topic section 351AV). By such selection, and the engagement of the "Images" tab 371P of the display section 371 of the adaptive display 307, image information is drawn from the information resource component 117B and displayed in the "Images" display section 391 of this embodiment. In the illustrated embodiment, the images that are displayed are displayed are echo images for the selected subject's aortic valve 391P. Advantageously, by providing the images or other information in the display section 391 that has already been organized for the aortic valve, the user is able to more efficiently enter observations in the Findings section for the Aortic valve 351AV and more quickly conduct a clinical study and complete a medical report for the subject.

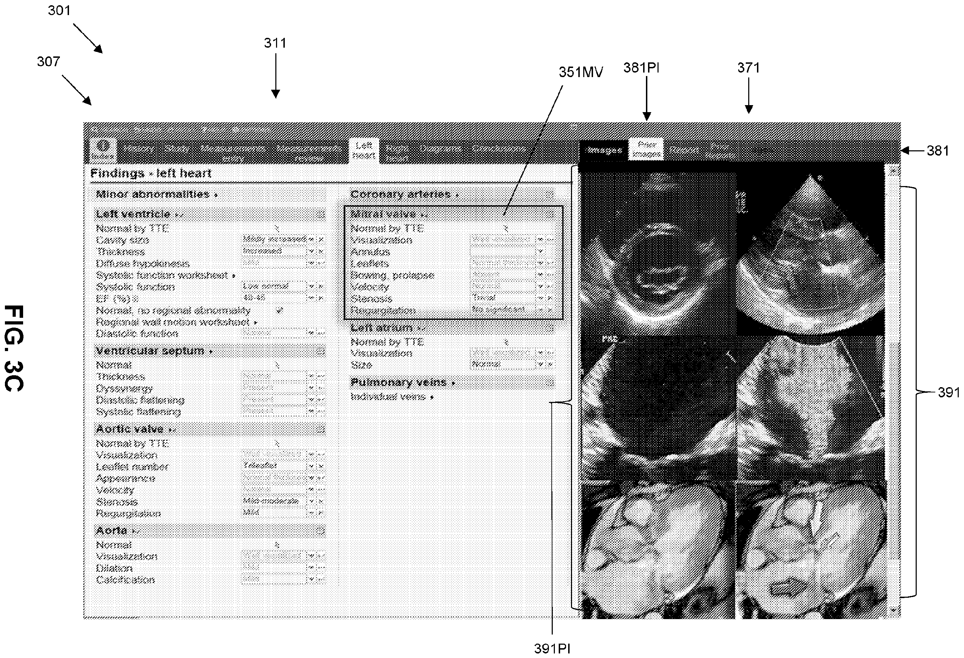

FIG. 3C illustrates the embodiment of the user interface screen 301 illustrated in FIG. 3A and FIG. 3B in which the topic identified by the heading "Mitral valve" 351MV is shown as being engaged by the user (shown by the shading around the "Mitral valve" topic section 351MV). By such selection, and the engagement of the "Prior Images" tab 371PI of the display section 371, image information is drawn from the information resource component 117B and displayed in the "Images" display section 391 of this embodiment. In the illustrated embodiment, the images that are displayed are a combination of echo images and MRI images for the selected subject's mitral valve 391PR. Advantageously, by providing such historical images or other information in the display section 391 that has already been organized for the mitral valve, the user is able to more efficiently enter observations in the Findings section for the Mitral valve 351MV and more quickly conduct a clinical study and complete a medical report for the subject.

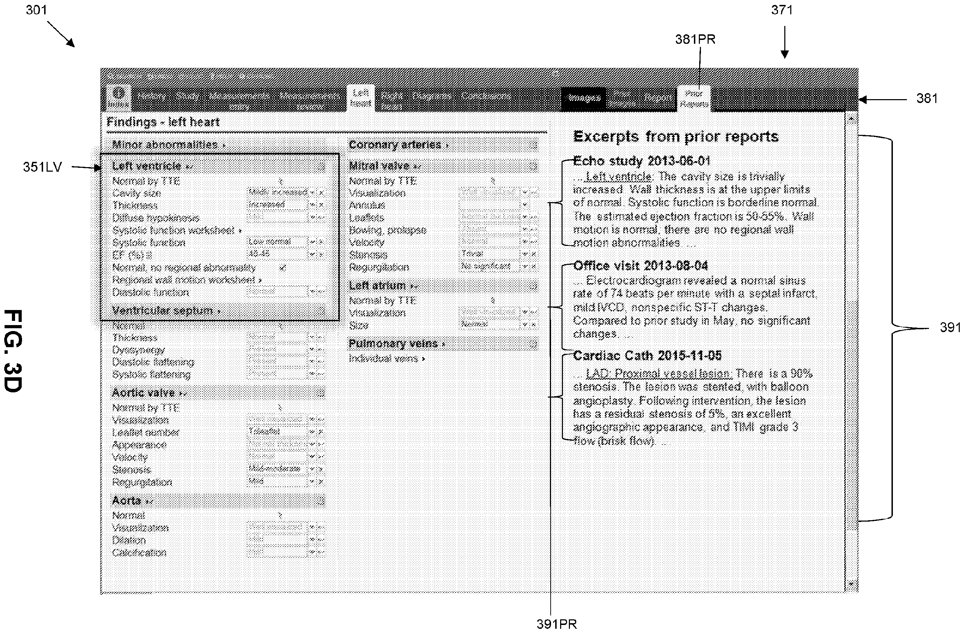

FIG. 3D illustrates the embodiment of the user interface screen 301 illustrated in FIG. 3A, FIG. 3B, and FIG. 3C in which the topic identified by the heading "Left ventricle" 351LV is shown as being engaged by the user (shown by the shading around the "Left ventricle" topic section 351LV). By such selection, and the engagement of the "Prior Reports" tab 371PR of the display section 371 of the adaptive display 307, report information is drawn from the information resource component 117B and displayed in the "Images" display section 391 of this embodiment. In the illustrated embodiment, information from prior reports 391PRare displayed. The illustrated embodiments of the adaptive display 307 provides an excerpt from a plurality of prior reports bearing a title and a date relevant to that excerpt, plus certain information from the relevant report. Advantageously, by providing such historical information in the display section 391 that has already been organized for the left ventricle, the user is able to more efficiently enter observations in the Findings section for the left ventricle 351LV and more quickly conduct a clinical study and complete a medical report for the subject.

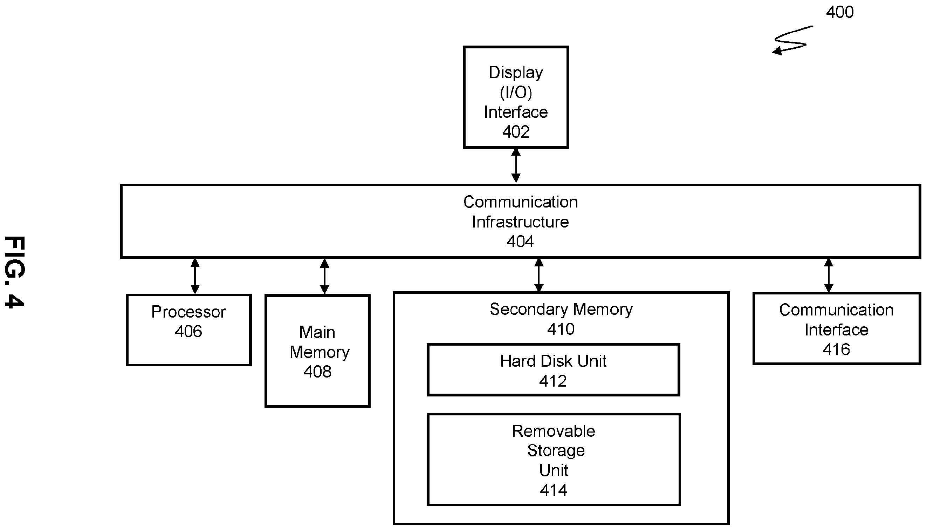

FIG. 4 illustrates a diagram of a system of which may be an embodiment of the present invention. Computer system 400 includes an input/output interface 402 connected to communication infrastructure 404 - such as a bus -, which forwards data such as graphics, text, and information, from the communication infrastructure 404 or from a frame buffer (not shown) to other components of the computer system 400. The input/output interface 402 may be, for example, a display device, a keyboard, touch screen, joystick, trackball, mouse, monitor, speaker, printer, Google Glass^{®} unit, web camera, any other computer peripheral device, or any combination thereof, capable of entering and/or viewing data.

Computer system 400 includes one or more processors 406, which may be a special purpose or a general-purpose digital signal processor configured to process certain information. Computer system 400 also includes a main memory 408, for example random access memory (RAM), read-only memory (ROM), mass storage device, or any combination thereof. Computer system 400 may also include a secondary memory 410 such as a hard disk unit 412, a removable storage unit 414, or any combination thereof. Computer system 400 may also include a communication interface 416, for example, a modem, a network interface (such as an Ethernet card or Ethernet cable), a communication port, a PCMCIA slot and card, wired or wireless systems (such as Wi-Fi, Bluetooth, Infrared), local area networks, wide area networks, intranets, etc.

It is contemplated that the main memory 408, secondary memory 410, communication interface 416, or a combination thereof, function as a computer usable storage medium, otherwise referred to as a computer readable storage medium, to store and/or access computer software including computer instructions. For example, computer programs or other instructions may be loaded into the computer system 400 such as through a removable storage device, for example, a floppy disk, ZIP disks, magnetic tape, portable flash drive, optical disk such as a CD or DVD or Blu-ray, Micro-Electro-Mechanical Systems (MEMS), nanotechnological apparatus. Specifically, computer software including computer instructions may be transferred from the removable storage unit 414 or hard disc unit 412 to the secondary memory 410 or through the communication infrastructure 404 to the main memory 408 of the computer system 400.

Communication interface 416 allows software, instructions and data to be transferred between the computer system 400 and external devices or external networks. Software, instructions, and/or data transferred by the communication interface 416 are typically in the form of signals that may be electronic, electromagnetic, optical or other signals capable of being sent and received by the communication interface 416. Signals may be sent and received using wire or cable, fiber optics, a phone line, a cellular phone link, a Radio Frequency (RF) link, wireless link, or other communication channels.

Computer programs, when executed, enable the computer system 400, particularly the processor 406, to implement the methods of the invention according to computer software including instructions.

The computer system 400 described may perform any one of, or any combination of, the steps of any of the methods according to the invention. It is also contemplated that the methods according to the invention may be performed automatically.

The computer system 400 of FIG. 4 is provided only for purposes of illustration, such that the invention is not limited to this specific embodiment. It is appreciated that a person skilled in the relevant art knows how to program and implement the invention using any computer system.

The computer system 400 may be a handheld device and include any small-sized computer device including, for example, a personal digital assistant (PDA), smart hand-held computing device, cellular telephone, or a laptop or netbook computer, hand held console or MP3 player, tablet, or similar hand held computer device, such as an iPad^{®}, iPad Touch^{®} or iPhone^{®}.

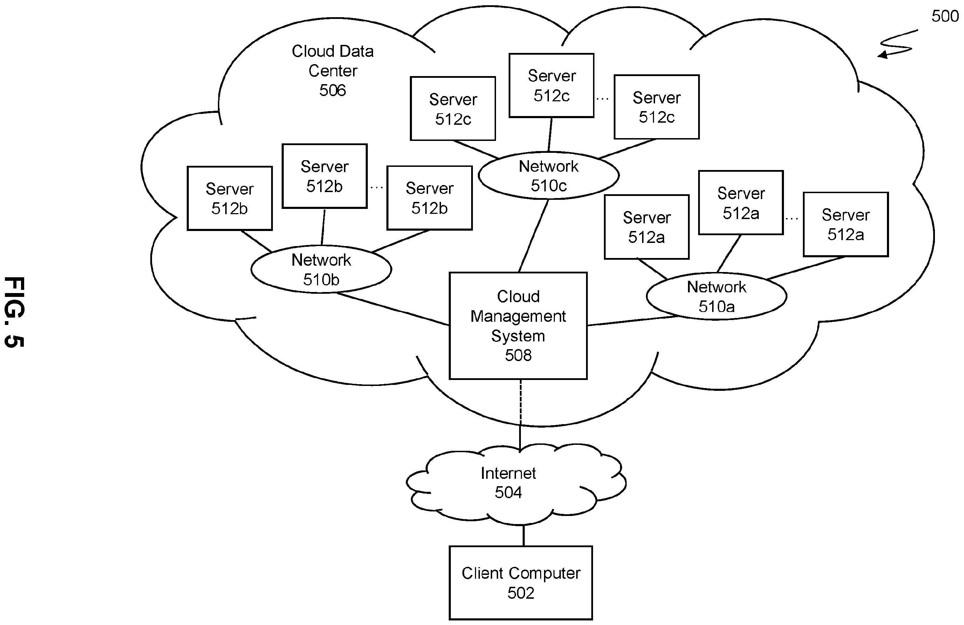

FIG. 5 illustrates an exemplary cloud computing system 500 that may be an embodiment of the present invention. The cloud computing system 500 includes a plurality of interconnected computing environments. The cloud computing system 500 utilizes the resources from various networks as a collective virtual computer, where the services and applications can run independently from a particular computer or server configuration making hardware less important.

Specifically, the cloud computing system 500 includes at least one client computer 502. The client computer 502 may be any device through the use of which a distributed computing environment may be accessed to perform the methods disclosed herein, for example, a traditional computer, portable computer, mobile phone, personal digital assistant, tablet to name a few. The client computer 502 includes memory such as random access memory (RAM), read-only memory (ROM), mass storage device, or any combination thereof. The memory functions as a computer usable storage medium, otherwise referred to as a computer readable storage medium, to store and/or access computer software and/or instructions.

The client computer 502 also includes a communications interface, for example, a modem, a network interface (such as an Ethernet card), a communications port, a PCMCIA slot and card, wired or wireless systems, etc. The communications interface allows communication through transferred signals between the client computer 502 and external devices including networks such as the Internet 504 and cloud data center 506. Communication may be implemented using wireless or wired capability such as cable, fiber optics, a phone line, a cellular phone link, radio waves or other communication channels.

The client computer 502 establishes communication with the Internet 504 - specifically to one or more servers - to, in turn, establish communication with one or more cloud data centers 506. A cloud data center 506 includes one or more networks 510a, 510b, 510c managed through a cloud management system 508. Each network 510a, 510b, 510c includes resource servers 512a, 512b, 512c, respectively. Servers 512a, 512b, 512c permit access to a collection of computing resources and components that can be invoked to instantiate a virtual machine, process, or other resource for a limited or defined duration. For example, one group of resource servers can host and serve an operating system or components thereof to deliver and instantiate a virtual machine. Another group of resource servers can accept requests to host computing cycles or processor time, to supply a defined level of processing power for a virtual machine. A further group of resource servers can host and serve applications to load on an instantiation of a virtual machine, such as an email client, a browser application, a messaging application, or other applications or software.

The cloud management system 508 can comprise a dedicated or centralized server and/or other software, hardware, and network tools to communicate with one or more networks 510a, 510b, 510c, such as the Internet or other public or private network, with all sets of resource servers 512a, 512b, 512c. The cloud management system 508 may be configured to query and identify the computing resources and components managed by the set of resource servers 512a, 512b, 512c needed and available for use in the cloud data center 506. Specifically, the cloud management system 508 may be configured to identify the hardware resources and components such as type and amount of processing power, type and amount of memory, type and amount of storage, type and amount of network bandwidth and the like, of the set of resource servers 512a, 512b, 512c needed and available for use in the cloud data center 506. Likewise, the cloud management system 508 can be configured to identify the software resources and components, such as type of Operating System (OS), application programs, and the like, of the set of resource servers 512a, 512b, 512c needed and available for use in the cloud data center 506.

The present invention is also directed to computer products, otherwise referred to as computer program products, to provide software to the cloud computing system 500. Computer products store software on any computer useable medium, known now or in the future. Such software, when executed, may implement the methods according to certain embodiments of the invention. Examples of computer useable mediums include, but are not limited to, primary storage devices (e.g., any type of random access memory), secondary storage devices (e.g., hard drives, floppy disks, CD ROMS, ZIP disks, tapes, magnetic storage devices, optical storage devices, Micro-Electro-Mechanical Systems (MEMS), nanotechnological storage device, etc.), and communication mediums (e.g., wired and wireless communications networks, local area networks, wide area networks, intranets, etc.). It is to be appreciated that the embodiments described herein may be implemented using software, hardware, firmware, or combinations thereof.

The cloud computing system 500 of FIG. 5 is provided only for purposes of illustration and does not limit the invention to this specific embodiment. It is appreciated that a person skilled in the relevant art knows how to program and implement the invention using any computer system or network architecture.

While the disclosure is susceptible to various modifications and alternative forms, specific exemplary embodiments of the invention have been shown by way of example in the drawings and have been described in detail. It should be understood, however, that there is no intent to limit the disclosure to the particular embodiments disclosed, but on the contrary, the intention is to cover all modifications, equivalents, and alternatives falling within the scope of the disclosure as defined by the appended claims.

## Claims

1. A computer-implemented method for preparing a medical report for a subject, the method comprising the following steps, carried out by a computer:
defining a selected clinical study for the subject (131a);
selecting a clinical concept ontology (131b) based on the selected clinical study to provide a template report (137B);
identifying, according to the selected clinical concept ontology, information relevant to the subject and relevant to the clinical study (121B);
processing the information relevant to the subject and relevant to the clinical study, according to the clinical concept ontology, to produce ontology-defined information, wherein the ontology-defined information comprises a plurality of images (123a);
deconstructing each image of the plurality by segregating each image into one or more image views, identifying an image modality used to produce each image, and isolating one or more structures within each image view (123b, 123c, 123d, 123e, 123f, 123g);
storing for each image the one or more image views, the image modality, and the one or more isolated structures (119a, 119b, 119n) in a database (117B);
forming an adaptive report by selecting from the database and adding to the template report two or more image views (123h), each image view from a different image modality, and the one or more isolated structures, according to an ontological structure of the template report (151); and
distributing the adaptive report to one or more users through a network for receipt in a computer system for display and use to prepare the medical report for the subject (171B).

2. The method according to claim 1, wherein the information includes image information (117B).

3. The method according to claim 1, wherein the information includes data information (117B).

4. The method according to claim 1, wherein said processing step further includes deconstructing data to develop data information (123f).

5. The method according to claim 1, wherein the information is drawn from third party sources.

6. The method according to claim 4, wherein said processing step further includes organizing and assembling said images and data according to the selected clinical concept ontology (115B).

7. The method according to claim 1, wherein said adaptive report including a findings tab section and an information tab section, said findings tab section and said information tab section each include one or more tabs organized according to one or more topics (321).

8. The method according to claim 7, further comprising an engagement component though which a user interacts with said adaptive report, wherein one or more images is output in response to engaging the one or more tabs of said information tab section (391).

## Patentansprüche

1. Computerimplementiertes Verfahren zum Vorbereiten eines medizinischen Berichtes für ein Subjekt, wobei das Verfahren die folgenden Schritte umfasst, welche durch einen Computer ausgeführt werden:
Definieren einer ausgewählten klinischen Studie für das Subjekt (131a);
Auswählen einer klinischen Konzept-Ontologie (131b) auf Grundlage der ausgewählten klinischen Studie, um einen Vorlagenbericht (137B) bereitzustellen;
Identifizieren, gemäß der ausgewählten klinischen Konzept-Ontologie, von Informationen, welche für das Subjekt relevant sind und für die klinische Studie (121B) relevant sind;
Verarbeiten der Informationen, welche für das Subjekt relevant sind und für die klinische Studie relevant sind, gemäß der klinischen Konzept-Ontologie, um Ontologiedefinierte Informationen zu erzeugen, wobei die Ontologie-definierten Informationen eine Mehrzahl von Bildern (123a) umfassen,
Zerlegen jedes Bildes der Mehrzahl durch Trennen jedes Bildes in eine oder mehrere Bildansichten, Identifizieren einer Bildmodalität, welche verwendet wird, um jedes Bild zu erzeugen, und Isolieren einer oder mehrerer Strukturen innerhalb jeder Bildansicht (123b, 123c, 123d, 123e, 123f, 123g);
Speichern, für jedes Bild, der einen oder der mehreren Bildansichten, der Bildmodalität und der einen oder der mehreren isolierten Strukturen (119a, 119b, 119n) in einer Datenbank (117B);
Bilden eines adaptiven Berichts, indem zwei oder mehr Bildansichten (123h), jede Bildansicht von einer verschiedenen Bildmodalität, und die eine oder die mehreren isolierten Strukturen, gemäß einer ontologischen Struktur des Vorlagenberichts (151), aus der Datenbank ausgewählt und zu dem Vorlagenbericht hinzugefügt werden; und
Verteilen des adaptiven Berichts an einen oder mehrere Benutzer durch ein Netzwerk für einen Empfang in einem Computersystem für eine Anzeige und eine Verwendung, um den medizinischen Bericht für das Subjekt (171B) vorzubereiten.

2. Verfahren nach Anspruch 1, wobei die Informationen Bildinformationen (117B) umfassen.

3. Verfahren nach Anspruch 1, wobei die Informationen Dateninformationen (117B) umfassen.

4. Verfahren nach Anspruch 1, wobei der Verarbeitungsschritt ferner ein Zerlegen von Daten umfasst, um Dateninformationen (123f) zu entwickeln.

5. Verfahren nach Anspruch 1, wobei die Informationen aus Quellen Dritter gezogen werden.

6. Verfahren nach Anspruch 4, wobei der Verarbeitungsschritt ferner ein Organisieren und Anordnen der Bilder und der Daten gemäß der ausgewählten klinischen Konzept-Ontologie (115B) umfasst.

7. Verfahren nach Anspruch 1, wobei der adaptive Bericht einen Befund-Registerabschnitt und einen Informationen-Registerabschnitt umfasst, wobei der Befund-Registerabschnitt und der Informationen-Registerabschnitt jeweils ein oder mehrere Register umfassen, welche gemäß einem oder mehreren Themen (321) organisiert sind.

8. Verfahren nach Anspruch 7, ferner umfassend eine Eingriffskomponente, durch welche ein Benutzer mit dem adaptiven Bericht interagiert, wobei in Reaktion auf ein Eingreifen mit dem einen oder den mehreren Registern des Informationen-Registerabschnitts (391) ein oder mehrere Bilder ausgegeben werden.

## Revendications

1. Procédé mis en oeuvre par ordinateur pour préparer un rapport médical pour un sujet, le procédé comprenant les étapes suivantes, réalisées par un ordinateur :
la définition d'une étude clinique sélectionnée pour le sujet (131a) ;
la sélection d'une ontologie de concept clinique (131b) sur la base de l'étude clinique sélectionnée pour fournir un modèle de rapport (137B) ;
l'identification, selon l'ontologie de concept clinique sélectionnée, d'information concernant le sujet et concernant l'étude clinique (121B) ;
le traitement de l'information concernant le sujet et concernant l'étude clinique, selon l'ontologie de concept clinique, pour produire des informations définies par l'ontologie, dans lequel les informations définies par l'ontologie comprennent une pluralité d'images (123a) ;
la déconstruction de chaque image de la pluralité par ségrégation de chaque image en une ou plusieurs vues d'image, l'identification d'une modalité d'image utilisée pour produire chaque image, et l'isolement d'une ou plusieurs structures dans chaque vue d'image (123b, 123c, 123d, 123e, 123f, 123g) ;
le stockage pour chaque image des une ou plusieurs vues d'image, de la modalité d'image, et des une ou plusieurs structures isolées (119a, 119b, 119n) dans une base de données (117B) ;
la formation d'un rapport adaptatif par sélection à partir de la base de données et l'ajout au modèle de rapport de deux vues d'image (123h) ou plus, chaque vue d'image à partir d'une modalité d'image différente, et les une ou plusieurs structures isolées, selon une structure anthologique du modèle de rapport (151) ; et
la distribution du rapport adaptatif à un ou plusieurs utilisateurs par l'intermédiaire d'un réseau pour réception dans un système informatique pour affichage et utiliser pour préparer le rapport médical pour le sujet (171B).

2. Procédé selon la revendication 1, dans lequel les informations comportent les informations d'image (117B).

3. Procédé selon la revendication 1, dans lequel les informations comportent des informations de données (117B).

4. Procédé selon la revendication 1, dans lequel ladite étape de traitement comporte en outre la déconstruction de données pour développer des informations de données (123f).

5. Procédé selon la revendication 1, dans lequel l'information est extraite de sources tierces.

6. Procédé selon la revendication 4, dans lequel ladite étape de traitement comporte en outre l'organisation et l'assemblage desdites images et données selon l'ontologie de concept clinique (115B) sélectionnée.

7. Procédé selon la revendication 1, dans lequel ledit modèle adaptatif comportant une section d'onglet de résultats d'examen et une section d'onglet d'information, ladite section d'onglet de résultats d'examen et ladite section d'onglet d'information comportent chacune un ou plusieurs onglets organisés selon un ou plusieurs thèmes (321).

8. Procédé selon la revendication 7, comprenant en outre un composant d'entrée en contact par l'intermédiaire duquel un utilisateur interagit avec ledit rapport adaptatif, dans lequel une ou plusieurs images sont sorties en réponse à l'entrée en contact avec les un ou plusieurs onglets de ladite section d'onglet d'information (391).
